# EUROPÄISCHE PATENTANMELDUNG

(11) **EP 3 444 029 A1**
(43) Veröffentlichungstag der Anmeldung: **20.02.2019**
(21) Anmeldenummer: 18188872.8
(22) Anmeldetag: 14.08.2018
(51) Int. Cl.: B01J 20/282, B01J 20/30, B01D 15/12, B01D 15/26, B01D 15/42, C07K 1/36, B01D 27/08

(54) **PROTEINREINIGUNGSVORRICHTUNG**

(30) Priorität: 17.08.2017 DE 102017007808
(71) Anmelder: Sartorius Stedim Biotech GmbH, 37079 Göttingen (DE)
(72) Erfinder: Pflanz, Karl, 37130 Gleichen (DE); Pickl, Andreas, 37133 Klein Schneen (DE); Linne, Holger, 37075 Göttingen (DE); Schröder-Tittmann, Kathrin, 37075 Göttingen (DE); Ransiek, Vanessa, 49205 Hasbergen (DE)
(74) Vertreter: Müller-Boré & Partner Patentanwälte PartG mbB

(57) **Zusammenfassung**

Die vorliegende Erfindung betrifft eine Vorrichtung zur Aufreinigung von Protein aus einem proteinhaltigen Fluid sowie ein Verfahren zur Aufreinigung von Protein aus einem proteinhaltigen Fluid und eine Verwendung der erfindungsgemäßen Vorrichtung zur Aufreinigung von Protein aus einem proteinhaltigen Fluid.

## Beschreibung

Die vorliegende Erfindung betrifft eine Vorrichtung zur Aufreinigung von Protein aus einem proteinhaltigen Fluid sowie ein Verfahren zur Aufreinigung von Protein aus einem proteinhaltigen Fluid und eine Verwendung der erfindungsgemäßen Vorrichtung zur Aufreinigung von Protein aus einem proteinhaltigen Fluid.

In der Biotechnologie werden häufig lebende Zellen zur Gewinnung großer Mengen von Proteinen eingesetzt. Dabei werden die Gene, die als Baupläne für die gewünschten Proteine dienen, in geeignete Plasmide kloniert und zur Expression in die Zellen gebracht. Das auf diese Weise intrazellulär produzierte Protein kann nach Zellaufschluss aus dem Zellplasma durch Aufreinigung isoliert werden. Wird das Protein von der Zelle in das umgebende Medium sekretiert, entfällt der Zellaufschluss.

Im Stand der Technik sind Isolations-/Aufreinigungsverfahren bekannt, die üblicherweise einem klassischen Ablauf folgen: Eine Suspension von Zellen, die das gewünschte Protein enthält, wird zentrifugiert und der Überstand wird verworfen. Das erhaltene Zellpellet wird in Puffer resuspendiert und die resuspendierten Zellen werden mechanisch oder chemisch aufgeschlossen. Anschließend wird, nach einem zweiten Zentrifugationsschritt zur Entfernung der Zelltrümmer, der Überstand mittels einer mit (gelartigem) Chromatographiematerial gefüllten Säule aufgetrennt, insbesondere unter Verwendung von Chromatographiematerialien, die das gewünschte Protein selektiv binden. Nach Abschluss des Chromatographievorgangs mit anschließendem Waschschritt kann das gebundene Protein mit Elutionspufferlösung von der Säule gelöst und aufgefangen werden.

Das vorstehende Verfahren ist jedoch mit einigen Nachteilen verbunden. Um angemessene Durchflussgeschwindigkeiten bei der Säulenchromatographie zu erzielen, wird diese oftmals im Vakuum bzw. mit Hilfe von Unterdruck durchgeführt. Dies erhöht jedoch den apparativen Aufwand, da ein Anschlusssystem zur Erzeugung des Unterdrucks vorhanden sein muss. Zudem wirkt sich Unterdruck negativ auf die aufzureinigende Proteinlösung aus, da diese bei Unterdruck oftmals aufschäumt und das zu klärende Protein ausfällt.

Bei größeren Mengen an aufzureinigendem Protein wird üblicherweise eine klassische FPLC-Anlage ("Fast Protein Liquid Chromatography"; Chromatographie unter Druck von üblicherweise 0,2 bis 10 bar) verwendet. Hierzu sind jedoch viele Einzelschritt wie eine vorausgehende Zentrifugation, Auftragung auf die Säule usw. nötig.

Des Weiteren muss gelartiges Chromatographiematerial zur Erhaltung seiner Stabilität unter Flüssigkeit und kühl gelagert werden. Dies führt jedoch zu einer erhöhten Gefahr der Verkeimung, insbesondere da sich derartiges Chromatographiematerial nicht mit einfachen Methoden sterilisieren lässt.

Zur Vermeidung des Einsatzes von Unterdruck wurden chromatographische Verfahren vorgeschlagen, bei denen die Durchflussgeschwindigkeit durch Zentrifugation erhöht wird. Aufgrund der benötigten Säulenlänge zur effektiven chromatischen Auftrennung eignen sich die sogenannten Spinsäulen jedoch nur zur Reinigung vor Proben mit einer geringen Fluidmenge. Handelsübliche Spinsäulen haben beispielsweise ein Maximalvolumen an aufnehmbarer, aufzureinigender Proteinlösung von 0,5 bis 2 mL.

Aufgrund der vorstehend beschriebenen Nachteile liegt der vorliegenden Erfindung die Aufgabe zu Grunde, eine apparativ einfache Vorrichtung bereitzustellen, die es ermöglicht, schnell (d.h. mit wenigen Verfahrensschritten), effektiv (d.h. eine hohe Ausbeute an gereinigtem Protein pro Zeiteinheit), zuverlässig und steril Protein aus einem proteinhaltigen Fluid aufzureinigen, sowie ein Verfahren, das diese Vorrichtung verwendet, und eine Verwendung der erfindungsgemäßen Vorrichtung.

Diese Aufgabe wird durch die in den Ansprüchen gekennzeichneten Ausführungsformen der vorliegenden Erfindung gelöst.

Insbesondere wird erfindungsgemäß eine Vorrichtung zum Aufreinigen von Protein aus einem proteinhaltigen Fluid bereitgestellt, umfassend
eine zylinderförmige Vorfiltereinheit (1), die mindestens einen Größenausschlussfilter (4) aufweist;
eine zylinderförmige Membranadsorbereinheit (2), die mindestens eine poröse Membran (5) mit funktionellen Bestandteilen aufweist, die das aufzureinigende Protein adsorbieren; und
einen zylinderförmigen Auffangbehälter (3); wobei der Auffangbehälter (3), die Membranadsorbereinheit (2) und die Vorfiltereinheit (1) derart ausgestaltet sind, dass der Auffangbehälter (3) zur Aufnahme der Membranadsorbereinheit (2) geeignet ist und die Membranadsorbereinheit (2) zur Aufnahme der Vorfiltereinheit (1) geeignet ist.

Erfindungsgemäß wird unter dem Begriff "proteinhaltiges Fluid" jegliches Fluid verstanden, das Protein enthält. Insbesondere eignen sich die erfindungsgemäße Vorrichtung und das erfindungsgemäße Verfahren zur Aufreinigung von proteinhaltiger Zellbrühe, d.h. einer Lösung bzw. Suspension, die Zellen und/oder Fragmente von chemisch oder mechanisch aufgeschlossene Zellen, welche das aufzureinigende Protein produziert haben, und das aufzureinigende Protein umfassen.

Das aufzureinigende Protein unterliegt erfindungsgemäß keiner besonderen Einschränkung. Beispielsweise können mit der erfindungsgemäßen Vorrichtung Proteine wie Antikörper oder getaggte (markierte) Proteine, beispielsweise Hisgetaggte (His-markierte) Proteine, aufgereinigt werden.

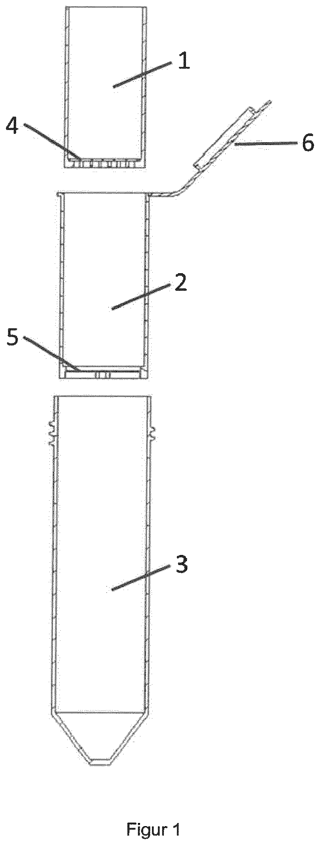

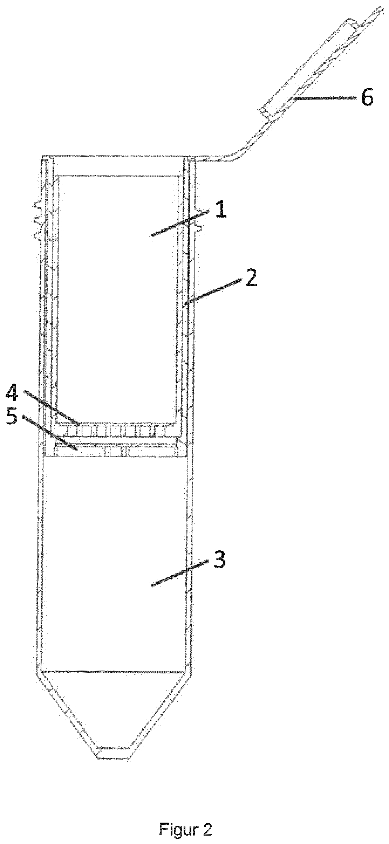

Die erfindungsgemäße Vorrichtung, die zur Aufreinigung von Protein mittels Zentrifugation geeignet ist, wird im Folgenden anhand einer in den Figuren 1 und 2 dargestellten bevorzugten Ausführungsform näher beschrieben. Figur 1 zeigt die Bauteile der erfindungsgemäßen Vorrichtung als Einzelteile. Figur 2 zeigt die erfindungsgemäße Vorrichtung in einem Zustand ("zusammengesteckter Zustand"), in dem sich die Vorfiltereinheit (1) vollständig in der Membranadsorbereinheit (2) befindet und die Membranadsorbereinheit (2) sich wiederum vollständig im Auffangbehälter (3) befindet. In diesem zusammengesteckten Zustand ist die erfindungsgemäße Vorrichtung geeignet für eine Zentrifugation zum Aufreinigen von Protein aus einem proteinhaltigen Fluid, welches sich vor der Zentrifugation in der Vorfiltereinheit (1) befindet.

Die erfindungsgemäße Vorrichtung umfasst eine zylinderförmige Vorfiltereinheit (1), eine zylinderförmige Membranadsorbereinheit (2) und einen zylinderförmigen Auffangbehälter (3). Erfindungsgemäß wird unter dem Begriff "zylinderförmig" verstanden, dass die Vorfiltereinheit (1), die Membranadsorbereinheit (2) und der Auffangbehälter (3) Hohlkörper sind, die durch zylinderförmige Wände begrenzt werden.

Das Material der zylinderförmigen Wände unterliegt keiner besonderen Einschränkung und es können beispielsweise alle gängigen Materialien für Zentrifugationsbehälter verwendet werden. Gemäß einer bevorzugten Ausführungsform umfasst das Material der zylinderförmigen Wände endotoxinfreie und gamma-sterilisierbare Kunststoffe wie beispielsweise Polypropylen, Polycarbonat, styrolhaltige Kunststoffe wie ABS (Acrylnitril-Butadien-Styrol), PEEK (Polyetheretherketon), Polyamid, Polyethylen, Polybutylen und PET (Polyethylenterephthalat).

Die Wanddicken der Vorfiltereinheit (1), der Membranadsorbereinheit (2) und des Auffangbehälters (3) unterliegen keiner besonderen Einschränkung. Gemäß einer bevorzugten Ausführungsform der vorliegenden Erfindung befinden sich die jeweiligen Wanddicken in einem Bereich von 0,25 mm bis 5 mm, besonders bevorzugt von 0,6 mm bis 2 mm.

Der Auffangbehälter (3), die Membranadsorbereinheit (2) und die Vorfiltereinheit (1) sind derart ausgestaltet, dass der Auffangbehälter (3) zur Aufnahme der Membranadsorbereinheit (2) geeignet ist und die Membranadsorbereinheit (2) zur Aufnahme der Vorfiltereinheit (1) geeignet ist. Erfindungsgemäß wird unter "zur Aufnahme geeignet" verstanden, dass ein Bauteil teilweise oder vollständig in ein anderes Bauteil eingeführt werden kann, d.h. sich teilweise oder vollständig im Zylinderinneren des anderen Bauteils befinden kann. Gemäß einer besonders bevorzugten Ausführungsform ist der Auffangbehälter (3) zur vollständigen Aufnahme der Membranadsorbereinheit (2) geeignet und ist die Membranadsorbereinheit (2) zur vollständigen Aufnahme der Vorfiltereinheit (1) geeignet, damit die erfindungsgemäße Vorrichtung mit einer Endkappe (6) verschlossen werden kann. Optional umfasst die erfindungsgemäße Vorrichtung zudem einen Klemmring, der die poröse Membran (5) in der Membranadsorbereinheit (2) festklemmt und gleichzeitig als Abstandshalter zwischen Membranadsorbereinheit (2) und Vorfiltereinheit (1) dient.

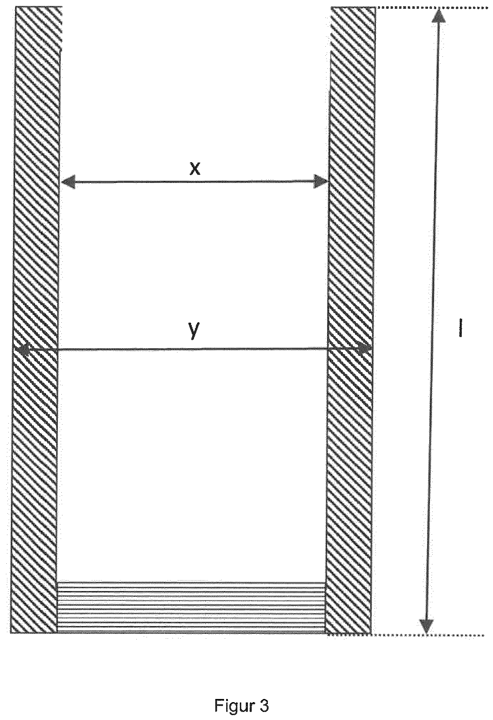

Erfindungsgemäß wird unter "Innenwandzylinderdurchmesser" der Durchmesser des Zylinders, gemessen von Innenwand zu Innenwand, verstanden ("x" in Figur 3), wohingegen der "Außenwandzylinderdurchmesser" der Durchmesser des Zylinders, gemessen von Außenwand zu Außenwandwand, ist ("y" in Figur 3). Damit der Auffangbehälter (3) zur Aufnahme der Membranadsorbereinheit (2) geeignet ist und damit die Membranadsorbereinheit (2) zur Aufnahme der Vorfiltereinheit (1) geeignet ist, ist der Innenwandzylinderdurchmesser des Auffangbehälters (3) erfindungsgemäß größer als der Außenwandzylinderdurchmesser der Membranadsorbereinheit (2), während der Innenwandzylinderdurchmesser der Membranadsorbereinheit (2) wiederum größer als der Außenwandzylinderdurchmesser der Vorfiltereinheit (1) ist. Gemäß einer bevorzugten Ausführungsform ist der Innenwandzylinderdurchmesser des Auffangbehälters (3) geringfügig, d.h. maximal 1 mm, größer als der Außenwandzylinderdurchmesser der Membranadsorbereinheit (2), und der Innenwandzylinderdurchmesser der Membranadsorbereinheit (2) ist geringfügig, d.h. maximal 1 mm, größer als der Außenwandzylinderdurchmesser der Vorfiltereinheit (1). Dadurch wird gewährleistet, dass einerseits die Bauteile passgenau eingeführt werden können und damit während einer Zentrifugation nicht aneinanderschlagen und dadurch beschädigt werden, und andererseits ein Maximalvolumen an proteinhaltigem Fluid vor einer Zentrifugation in der Vorfiltereinheit (1) aufgenommen werden kann.

Der Außenwandzylinderdurchmesser des Auffangbehälters (3) unterliegt keiner besonderen Einschränkung. Bevorzugt ist der Außenwandzylinderdurchmesser des Auffangbehälters (3) der erfindungsgemäßen Vorrichtung derart ausgewählt, dass die Vorrichtung in einer handelsüblichen Zentrifuge verwendet werden kann.

Die Längen ("I" in Figur 3) der zylinderförmigen Vorfiltereinheit (1), der zylinderförmigen Membranadsorbereinheit (2) und des zylinderförmigen Auffangbehälter (3) unterliegen keiner besonderen Einschränkung. Bevorzugt ist die Gesamtlänge der erfindungsgemäßen Vorrichtung im "zusammengesteckten Zustand" der Figur 2 derart ausgewählt, dass die Vorrichtung in einer handelsüblichen Zentrifuge verwendet werden kann.

Gemäß einer bevorzugten Ausführungsform der vorliegenden Erfindung sind die Längen der Vorfiltereinheit (1) und der Membranadsorbereinheit (2) ungefähr halb so groß wie die Länge des Auffangbehälters (3), wobei die Länge der Vorfiltereinheit (1) geringfügig kleiner als die Länge der Membranadsorbereinheit (2) ist. In diesem Fall kann im "zusammengesteckten Zustand" der Figur 2 der erfindungsgemäßen Vorrichtung die Vorfiltereinheit (1) ungefähr dasselbe Volumen an Fluid aufnehmen wie der Bereich des Auffangbehälters (3), der nicht von der Vorfiltereinheit (1) und der Membranadsorbereinheit (2) in Anspruch genommen wird. Dadurch wird gewährleistet, dass bei einem Zentrifugationsvorgang eine Maximalmenge an proteinhaltigem Fluid eingesetzt werden kann, ohne dass am Ende der Zentrifugation ein Fluidrückstand in der Vorfiltereinheit (1) verbleibt. Gemäß einer besonders bevorzugten Ausführungsform der vorliegenden Erfindung sind die Vorfiltereinheit (1) und der Auffangbehälter (3) derart ausgestaltet, dass der Auffangbehälter (3) zur Aufnahme von 1 bis 50 mL Fluid (beispielsweise von 10 bis 40 mL) und die Vorfiltereinheit (1) zur Aufnahme von 0,5 bis 25 mL Fluid geeignet sind. Dadurch wird gewährleistet, dass bei einem Zentrifugationsvorgang der erfindungsgemäßen Vorrichtung im "zusammengesteckten Zustand" der Figur 2 in einer handelsüblichen Standardzentrifuge eine Maximalmenge an proteinhaltigem Fluid eingesetzt werden kann, ohne dass am Ende der Zentrifugation ein Fluidrückstand in der Vorfiltereinheit (1) verbleibt.

Die zylinderförmige Vorfiltereinheit (1) der erfindungsgemäßen Vorrichtung ist geeignet, das proteinhaltige Fluid vor einer Zentrifugation aufzunehmen, und weist mindestens einen Größenausschlussfilter (4) auf. Der mindestens eine Größenausschlussfilter (4) hält vorteilhafterweise Zellreste und andere Bestandteile des proteinhaltigen Fluids bei einer Zentrifugation der erfindungsgemäßen Vorrichtung im zusammengesteckten Zustand zurück, welche entweder die poröse Membran (5) verblocken und damit zur Adsorption des Proteins unbrauchbar machen würden oder die eluierte Proteinlösung verunreinigen würden. Dadurch kann vorteilhafterweise in der vorliegenden Erfindung ein proteinhaltiges Fluid zur Aufreinigung eingesetzt werden, welches eine Zellbrühe ist, die keiner weiteren Vorreinigung/Filtration ausgesetzt wurde.

Größenausschlussfilter (4) im Sinne der vorliegenden Erfindung sind Tiefenfilter oder Mikrofilter und Ultrafilter, wie in Pure Appl. Chem., 1996, 68, 1479 definiert. Dabei ist eine entsprechende Mikrofiltration als ein druckgetriebenes Membran-basiertes Trennungsverfahren definiert, in welchem Teilchen und gelöste Makromoleküle größer als 0,1 µm zurückgehalten werden. Die Ultrafiltration wird hingegen als ein druckgetriebenes Membran-basiertes Trennungsverfahren definiert, in welchem Teilchen und gelöste Makromoleküle kleiner als 0,1 µm und größer als etwa 2 nm zurückgehalten werden. Als Material für Größenausschlussfilter kommen gängige Membranen und Filtermaterialien entweder polymerer Natur oder anorganischer Zusammensetzung (z.B. Glasfaser) in Frage, beispielsweise Polymere aus Cellulose, Cellulosederivaten wie -acetat und -nitrat, Polypropylen, Polyethylen, Polycarbonat, Polyetherketonen, Polyvinylidenfluorid, Polysulfonen, Polyethersulfonen, aromatischen und aliphatischen Polyamiden, Keramiken wie Al₂O₃, Silikate, Nitride, Boride und Mischungen dieser Stoffe in Frage, d.h. Materialien, aus denen sich poröse, mechanisch integre Formkörper herstellen lassen. Auch gängige Filterhilfsmittel wie beispielsweise Kieselgur können als Abtrennungshilfe verwendet werden.

Gemäß einer bevorzugten Ausführungsform ist die Größenausschlussgrenze (90% Cut-off) des Größenausschlussfilters (4) der erfindungsgemäßen Vorrichtung größer als 0,2 µm, damit sich keine partikulären Bestandteile in der nachfolgenden porösen Membran (5) festsetzen können. Gemäß einer bevorzugten Ausführungsform ist die Größenausschlussgrenze des Größenausschlussfilters (4) in einem Bereich von 0,05 µm bis 200 µm, besonders bevorzugt von 0,1 µm bis 10 µm. Die Dicke des Größenausschlussfilters (4) unterliegt erfindungsgemäß keiner besonderen Einschränkung. Gemäß einer bevorzugten Ausführungsform ist die Dicke in einem Bereich von 50 µm bis 2 cm, bevorzugt von 100 µm bis 0,5 cm.

Erfindungsgemäß ist der mindestens eine Größenausschlussfilter (4) senkrecht zur Längsrichtung ("I" in Figur 3) der zylinderförmigen Vorfiltereinheit (1) angeordnet. Die Position des Größenausschlussfilters (4) in der Längsrichtung unterliegt keiner besonderen Einschränkung. Gemäß einer bevorzugten Ausführungsform ist der mindestens eine Größenausschlussfilter (4) an einem Ende der zylinderförmigen Vorfiltereinheit (1) angeordnet, insbesondere an dem Ende der Vorfiltereinheit (1), das im "zusammengesteckten Zustand" der Vorrichtung in Richtung der Membranadsorbereinheit (2) weist, wie in den Figuren 1 und 2 gezeigt, da dadurch gewährleistet wird, dass ein Maximalvolumen an proteinhaltigem Fluid vor einer Zentrifugation in der Vorfiltereinheit (1) aufgenommen werden kann.

Gemäß einer Ausführungsform der vorliegenden Erfindung weist die zylinderförmige Vorfiltereinheit (1) zwei oder mehr Größenausschlussfilter (4) auf, die vorzugsweise benachbart angeordnet sind. Bei dieser Ausführungsform ist vorteilhafterweise ein Gradient möglich, beispielsweise ein grober Größenausschlussfilter (beispielsweise ein Tiefenfilter), gefolgt von einem feineren Größenausschlussfilter, was vorteilhafterweise zu einer hohen Aufnahmekapazität und einem hohen Durchsatz führt.

Gemäß einer besonders bevorzugten Ausführungsform weist die zylinderförmige Vorfiltereinheit (3) drei Größenausschlussfilter (4) auf, die vorzugsweise benachbart angeordnet sind. Der erste der drei Größenausschlussfilter (4), der am nächsten zum Ende der der Vorfiltereinheit (1) angeordnet ist, das im "zusammengesteckten Zustand" der Vorrichtung nicht in Richtung der Membranadsorbereinheit (2) weist, ist dabei vorzugsweise eine Glasfaserschicht mit einer Durchschnittsporengröße von beispielsweise 1,5 µm bis 3,25 µm. Derartige Glasfaserschichten sind kommerziell erhältlich, beispielsweise bei der Firma Sartorius Stedim Biotech GmbH. Der zweite der drei Größenausschlussfilter (4), der zwischen dem ersten und dritten Größenausschlussfilter gelegen ist, ist dabei vorzugsweise eine Glasfaserschicht mit einer Durchschnittsporengröße von beispielsweise 0,8 µm bis 1,4 µm. Derartige Glasfaserschichten sind kommerziell erhältlich, beispielsweise bei der Firma Sartorius Stedim Biotech GmbH. Der dritte der drei Größenausschlussfilter (4), der am nächsten zum Ende der der Vorfiltereinheit (1) angeordnet ist, das im "zusammengesteckten Zustand" der Vorrichtung in Richtung der Membranadsorbereinheit (2) weist, ist dabei vorzugsweise eine Polyethersulfonmembran (PES-Membran), insbesondere eine stark asymmetrische PES-Membran, beispielsweise mit einer Durchschnittsporengröße von 0,15 µm bis 0,3 µm. Die PES-Membran ist vorzugsweise gesiegelt, um zu verhindern, dass das proteinhaltige Fluid am Rande der Membran ungefiltert vorbeilaufen kann. Derartige PES-Membranen sind kommerziell erhältlich, beispielsweise bei der Firma Sartorius Stedim Biotech GmbH (Materialtyp "15427EP"). Gemäß einer besonders bevorzugten Ausführungsform sind die drei Größenausschlussfilter (4) benachbart an dem Ende der Vorfiltereinheit (1), das im "zusammengesteckten Zustand" der Vorrichtung in Richtung der Membranadsorbereinheit (2) weist, wie in den Figuren 1 und 2 gezeigt, angeordnet.

Die zylinderförmige Membranadsorbereinheit (2) der erfindungsgemäßen Vorrichtung weist mindestens eine poröse Membran (5) mit funktionellen Bestandteilen auf, d.h. mit chemischen Gruppen, die das aufzureinigende Protein (vorzugsweise selektiv) adsorbieren. Vorteilhafterweise bindet die poröse Membran (5) das aufzureinigende Protein bei konvektivem Fluss. Die funktionellen Bestandteile bzw. die chemischen Gruppen können entweder originär in/auf der porösen Membran (5) vorhanden sein oder durch dem Fachmann bekannte Verfahren nachträglich in/auf die poröse Membran (5) eingeführt worden sein. Erfindungsgemäß werden unter dem Begriff "chemische Gruppen" sowohl Gruppen von Atomen/Ionen/Molekülen als auch einzelne Atome bzw. Ionen verstanden, die in der Lage sind, das aufzureinigende Protein zu adsorbieren. Derartige funktionelle Bestandteile bzw. chemische Gruppen und auch deren Einführung in/auf eine poröse Membran sind dem Fachmann bekannt und werden je nach Art des aufzureinigenden Proteins eingesetzt. Als Beispiele für funktionelle Bestandteile bzw. chemische Gruppen der porösen Membran (5) zur Adsorption des aufzureinigenden Proteins können unter anderem Ni²⁺, Cu²⁺, Protein A, Streptadivin oder Derivate und Amylose genannt werden. Gemäß einer bevorzugten Ausführungsform umfassen die chemischen Gruppen Ni²⁺ und/oder Cu²⁺ - Ionen, die sich zur Adsorption von His-getaggten Proteinen eignen.

Die poröse Membran (5) in der Membranadsorbereinheit (2) der vorliegenden Erfindung kann entweder einlagig sein oder aus zwei oder mehr Membranlagen aufgebaut sein. Je mehr Membranlagen in der Membranadsorbereinheit (2) vorhanden sind, desto höher ist die totale Bindekapazität der Membranadsorbereinheit (2). Jedoch wird bei Verwendung zu vieler Membranladen das Elutionsvolumen zu hoch. Demzufolge ist die poröse Membran (5) vorzugsweise aus 3 bis 30 Membranlagen aufgebaut, besonders bevorzugt aus 5 bis 25 Membranlagen. Das Gerüstmaterial der porösen Membran (5) unterliegt keiner besonderen Einschränkung, und es können beispielsweise Membranen aus Cellulose-Hydrat/-Ester/-Acetat, Cellulose-Nanofasern, Nylon-Nanofasern, Polyethylenterephthalat (PET), Poly(oxy-1,4-phenylsulfonyl-1,4-phenyl), Polyamiden, Polyestern, Polyvinylidenfluorid (PVDF), Polyethylen (PE), Polypropylen (PP) und/oder Hydrogel-modifizierte Membranen verwendet werden.

Die Dicke der porösen Membran (5) unterliegt erfindungsgemäß keiner besonderen Einschränkung. Gemäß einer bevorzugten Ausführungsform ist die Dicke in einem Bereich von 50 µm bis 2 cm, bevorzugt von 100 µm bis 500 µm. Die Porengröße der porösen Membran (5) unterliegt erfindungsgemäß keiner besonderen Einschränkung. Gemäß einer bevorzugten Ausführungsform ist die Porengröße in einem Bereich von 0,1 µm bis 10 µm. Ist die poröse Membran (5) aus zwei oder mehr Membranlagen aufgebaut, so können diese Membranlagen voneinander unterschiedliche oder gleiche Membrandicken und/oder Porengrößen aufweisen.

Erfindungsgemäß ist die poröse Membran (5) senkrecht zur Längsrichtung ("I" in Figur 3) der zylinderförmigen Membranadsorbereinheit (2) angeordnet. Die Position der porösen Membran (5) in der Längsrichtung unterliegt keiner besonderen Einschränkung. Gemäß einer bevorzugten Ausführungsform ist die poröse Membran (5) an einem Ende der zylinderförmigen Membranadsorbereinheit (2) angeordnet, insbesondere an dem Ende der Membranadsorbereinheit (2), das im "zusammengesteckten Zustand" der Vorrichtung in Richtung des Auffangbehälters (3) weist, wie in den Figuren 1 und 2 gezeigt, da dadurch gewährleistet wird, dass eine möglichst große Vorfiltereinheit (1) und damit auch ein möglichst großes Volumen an in der Vorfiltereinheit (1) befindlichem Fluid in der Membranadsorbereinheit (2) aufgenommen werden können.

Vorteilhafterweise kann die erfindungsgemäße poröse Membran (5), im Gegensatz zu im Stand der Technik oftmals verwendeten Chromatographiemateralien wie in Säulen gepackte Gele, trocken (d.h. nicht unter Flüssigkeit) und bei Raumtemperatur gelagert werden, ohne dass die poröse Membran (5) ihre Stabilität verliert. Dadurch, dass sich die erfindungsgemäß verwendete poröse Membran (5) mit einfachen Methoden sterilisieren lässt (beispielsweise durch Bestrahlung mit gamma-Strahlung), kann vorteilhafterweise ohne hohen Aufwand eine Verkeimung vermieden werden.

Gemäß einer bevorzugten Ausführungsform umfasst die erfindungsgemäße Vorrichtung weiter ein Flussbeschränkungsmittel. Erfindungsgemäß wird unter dem Begriff "Flussbeschränkungsmittel" ein Bauteil verstanden, dass derart ausgestaltet ist, dass bei Zentrifugation der Vorrichtung die Verweildauer des proteinhaltigen Fluids in der mindestens einen porösen Membranlage erhöht wird. Dies führt vorteilhafterweise zu einer verbesserten Adsorption des aufzureinigenden Proteins an der porösen Membran (5) der zylinderförmigen Membranadsorbereinheit (2).

Gemäß einer besonders bevorzugten Ausführungsform ist das Flussbeschränkungsmittel in der zylinderförmigen Membranadsorbereinheit (2) angeordnet, insbesondere benachbart zur porösen Membran (5), insbesondere auf der Seite der porösen Membran (5), die im "zusammengesteckten Zustand" der Vorrichtung in Richtung des Auffangbehälters (3) weist. Gemäß der am meisten bevorzugten Ausführungsform ist das Flussbeschränkungsmittel an einem Ende der Membranadsorbereinheit (2) angeordnet, und die poröse Membran (5) ist direkt dem Flussbeschränkungsmittel benachbart in Richtung Zylinderzentrum in Längsrichtung der zylinderförmigen Membranadsorbereinheit (2) angeordnet. So wird einerseits gewährleistet, dass eine verbesserte Adsorption des aufzureinigenden Proteins an der porösen Membran (5) aufgrund der längeren Verweildauer des proteinhaltigen Fluids vorliegt, und andererseits gewährleistet, dass eine möglichst große Vorfiltereinheit (1) und damit auch ein möglichst großes Volumen an in der Vorfiltereinheit (1) befindlichem Fluid in der Membranadsorbereinheit (2) aufgenommen werden können.

Der Aufbau des Flussbeschränkungsmittels unterliegt keiner besonderen Einschränkung, solange das Flussbeschränkungsmittel dazu geeignet ist, bei Zentrifugierung der Vorrichtung die Verweildauer des proteinhaltigen Fluids in der porösen Membran (5) zu erhöhen. Beispiele für Flussbeschränkungsmittel sind Membranen, Filter, Ultrafilter und Mikrofilter (beispielsweise 0,1 µm Porengröße mit maximal 500 kDa, so dass keine Retention des Proteins eintritt).

Gemäß einer bevorzugten Ausführungsform der vorliegenden Erfindung ist das Flussbeschränkungsmittel mindestens eine Membran, insbesondere eine Membran, die eine geringere Porosität als die mindestens eine poröse Membran (5) der Membranadsorbereinheit (2) aufweist und somit die Flussgeschwindigkeit des Fluids verringert. Als Membranmaterialien können dabei beispielsweise die vorstehend aufgeführten Materialien betreffend die poröse Membran (5) eingesetzt werden. Die Dicke der Membran unterliegt erfindungsgemäß keiner besonderen Einschränkung. Gemäß einer bevorzugten Ausführungsform ist die Dicke in einem Bereich von 50 µm bis 3 cm, bevorzugt von 100 µm bis 2 cm. Die Porengröße der Membran unterliegt erfindungsgemäß keiner besonderen Einschränkung. Gemäß einer bevorzugten Ausführungsform ist die Porengröße in einem Bereich von 0,05 µm bis zur Porengröße der porösen Membran (5). Umfasst das Flussbeschränkungsmittel zwei oder mehr Membranen, so können diese Membranen voneinander unterschiedliche oder gleiche Membrandicken und/oder Porengrößen aufweisen.

Der zylinderförmige Auffangbehälter (3) der erfindungsgemäßen Vorrichtung unterliegt keiner besonderen Einschränkung, solange der Auffangbehälter (3) an einem Ende verschlossen ist und somit das Filtrat auffangen kann. Das geschlossene Ende des zylinderförmigen Auffangbehälters (3) kann sich beispielsweise zu einem Kegelstumpf verjüngen, wie in den Figuren 1 und 2 dargestellt.

Gemäß einer Ausführungsform umfasst die erfindungsgemäße Vorrichtung eine Endkappe (6), mit der sich die Vorfiltereinheit (1) und/oder die Membranadsorbereinheit (2) reversibel verschließen lässt. Dadurch wird gewährleistet, dass bei einer Zentrifugation der erfindungsgemäßen Vorrichtung im "zusammengesteckten Zustand" kein Fluid aus der Vorrichtung austritt. Die Endkappe (6) kann entweder ein separates Bauteil sein oder an der Vorfiltereinheit (1), der Membranadsorbereinheit (2) oder dem Auffangbehälter (3) beweglich befestigt sein, beispielsweise über Filmscharniere. Gemäß einer bevorzugten Ausführungsform ist die Endkappe (6) an dem der porösen Membran (5) / optionales Flussbeschränkungsmittel entgegengesetzten Ende der Membranadsorbereinheit (2) befestigt, wie in den Figuren 1 und 2 dargestellt.

Ein weiterer Aspekt der vorliegenden Erfindung betrifft ein Verfahren zur Aufreinigung von Protein aus einem proteinhaltigen Fluid, umfassend die Schritte
(a) Bereitstellen der erfindungsgemäßen Vorrichtung zur Aufreinigung von Protein aus einem proteinhaltigen Fluid, wobei sich die Vorfiltereinheit (1) in der Membranadsorbereinheit (2) befindet und sich die Membranadsorbereinheit (2) in dem Auffangbehälter (3) befindet;
(b) Befüllen der Vorfiltereinheit (1) mit proteinhaltigem Fluid;
(c) Zentrifugieren der Vorrichtung, wobei die poröse Membran (5) verblockende Bestandteile des proteinhaltigen Fluids von dem mindestens einen Größenausschlussfilter (4) zurückgehalten werden, das zu reinigende Protein an der porösen Membran (5) der Membranadsorbereinheit (2) adsorbiert und sich ein Filtrat in dem Auffangbehälter (3) sammelt;
(d) Verwerfen des im Auffangbehälter (3) befindlichen Filtrats;
(f) Befüllen der Membranadsorbereinheit (2) mit Elutionspufferlösung;
(g) Eluieren des an der porösen Membran (5) adsorbierten Proteins mit der Elutionspufferlösung in den Auffangbehälter (3) zum Erhalt des aufgereinigten Proteins.

Im Schritt (a) des erfindungsgemäßen Verfahrens wird die vorstehend beschriebene Vorrichtung bereitgestellt, wobei sich die Vorrichtung im "zusammengesteckten Zustand" wie in Figur 2 dargestellt befindet, d.h. die Vorfiltereinheit (1) befindet sich in der Membranadsorbereinheit (2) und die Membranadsorbereinheit (2) befindet sich in dem Auffangbehälter (3).

Im Schritt (b) des erfindungsgemäßen Verfahrens wird die Vorfiltereinheit (1) mit proteinhaltigem Fluid befüllt. Vorteilhafterweise kann als proteinhaltiges Fluid eine Zellbrühe verwendet werden (entweder mit aufgeschlossenen oder mit intakten Zellen), die keiner weiteren Vorreinigung/Filtration wie im Stand der Technik üblich ausgesetzt wurde, und daher Zellreste und andere Bestanteile, die potentiell die poröse Membran (5) (oder auch andere klassische Chromatographiematerialien) verblocken könnten, enthält. Dies ist deshalb möglich, da die erfindungsgemäße Vorrichtung in ihrer modularen Bauweise auch mindestens einen Größenausschlussfilter (4) in der Vorfiltereinheit (1) aufweist, welcher die poröse Membran (5) vor Verblockung schützt.

Im Schritt (c) des erfindungsgemäßen Verfahrens wird die mit proteinhaltiger Lösung befüllte Vorrichtung zentrifugiert. Dabei werden potentiell die poröse Membran (5) verblockende Bestandteile des proteinhaltigen Fluids von dem mindestens einen Größenausschlussfilter (4) zurückgehalten, während das aufzureinigende Protein den Größenausschlussfilter (4) ungehindert passiert. Das aufzureinigende Protein adsorbiert (selektiv) auf der dem Größenausschlussfilter (4) folgenden, porösen Membran (5) der Membranadsorbereinheit (2), während sich das vom Protein getrennte Filtrat im Auffangbehälter (3) sammelt.

Die Zentrifugationszeit und -geschwindigkeit im Schritt (c) unterliegt keiner besonderen Einschränkung und kann problemlos von einem Fachmann so ausgewählt werden, dass das zu Beginn der Zentrifugation in der Vorfiltereinheit (1) befindliche Fluid vollständig durch den Größenausschlussfilter (4) und die poröse Membran (5) bewegt wird und sich als Filtrat im Auffangbehälter (3) sammelt. Zudem ist dem Fachmann bekannt, dass die Zentrifugationsgeschwindigkeit nicht zu hoch sein sollte, da in diesem Fall eine vollständige Adsorption des aufzureinigenden Proteins an der porösen Membran (5) nicht gewährleistet werden kann.

Im Schritt (d) des erfindungsgemäßen Verfahrens werden die Vorfiltereinheit (1) und das im Auffangbehälter (3) befindliche Filtrat verworfen. Unter "Verwerfen" wird erfindungsgemäß verstanden, dass die Vorfiltereinheit (1) entweder entsorgt wird oder neu aufbereitet (gereinigt) wird, um für ein weiteres Aufreinigungsverfahren wiederverwendet werden zu können. Das Filtrat wird ebenso entweder entsorgt, oder kann optional einer erneuten Aufreinigung zugeführt werden, um noch gegebenenfalls geringfügig vorhandene Reste an zu reinigendem Protein im Filtrat ebenso zu isolieren. Die erneute Aufreinigung kann beispielsweise direkt nochmal über dieselbe Membran (5) erfolgen, oder es kann eine neue erfindungsgemäße Vorrichtung dazu verwendet werden.

Nach dem Schritt (d) umfasst das erfindungsgemäße Verfahren optional den Schritt (e). Im optionalen Schritt (e) wird die Membranadsorbereinheit (2) mit einer Waschpufferlösung befüllt, die erfindungsgemäße Vorrichtung ohne die verworfene Vorfiltereinheit (1) wird zentrifugiert, so dass sich die Waschpufferlösung in dem Auffangbehälter (3) sammelt. Anschließend wird die im Auffangbehälter (3) befindliche Waschpufferlösung verworfen. Der optionale Schritt (e) kann einmal oder auch mehrmals durchgeführt werden und entfernt vorteilhafterweise an die poröse Membran (5) ungebundene und unspezifisch gebundene Bestandteile des ursprünglich proteinhaltigen Fluids. Waschpufferlösungen sind dem Fachmann bekannt und es kann beispielsweise Puffer mit dem gewünschten pH-Wert und der gewünschten Salzkonzentration als Waschpufferlösung verwendet werden.

Im Schritt (f) des erfindungsgemäßen Verfahrens, der entweder nach dem Schritt (d) oder dem optionalen Schritt (e) erfolgt, wird die Membranadsorbereinheit mit Elutionspufferlösung befüllt. Der Fachmann kann in Abhängigkeit des aufzureinigenden Proteins und der verwendeten porösen Membran (5) eine Elutionspufferlösung auswählen, die in der Lage ist, das adsorbierte Protein von der porösen Membran (5) zu eluieren, beispielsweise einen Waschpuffer mit kompetitivem Molekül (z.B. Imidazol, Biotin oder Maltose), welches das Protein von der porösen Membran (5) verdrängen kann. Alternativ kann auch ein Elutionspuffer mit niedrigem pH-Wert verwendet werden.

Im Schritt (g) des erfindungsgemäßen Verfahrens wird das an der porösen Membran (5) adsorbierte Protein mit der Elutionspufferlösung in den Auffangbehälter (3) eluiert. Dabei wird das aufgereinigte Protein als Filtrat im Auffangbehälter (3) erhalten. Vorzugsweise erfolgt die Eluierung unter Zentrifugation.

Da das erfindungsgemäße Verfahren die nötige Durchflussgeschwindigkeit zur Aufreinigung des Proteins mittels Zentrifugation erzielt, ist ein Anlegen von Unterdruck bzw. Vakuum nicht nötig. Somit können vorteilhafterweise sämtliche Schritte des erfindungsgemäßen Verfahrens unter Standardatmosphärendruck durchgeführt werden.

Ein weiterer Aspekt der vorliegenden Erfindung betrifft die Verwendung der erfindungsgemäßen Vorrichtung zur Aufreinigung von Protein aus einem proteinhaltigem Fluid. Gemäß einer bevorzugten Ausführungsform ist das zu reinigende Protein ein His-getaggtes (His-markiertes) Protein oder ein Antikörper (beispielsweise mbp-getaggt oder strep-getaggt).

Die erfindungsgemäße Vorrichtung und das erfindungsgemäße Verfahren erlauben es, vorteilhafterweise in nur einem Zentrifugationsschritt gleichzeitig sowohl eine Größenausschlussfiltration als auch eine Proteinadsorption durchzuführen, um ein Zielprotein aufzureinigen. Dadurch ist es möglich, Protein aus ungeklärter Zellbrühe aufzureinigen, so dass vorteilhafterweise keine Vor-Filtrationsschritte notwendig sind. Da bei der erfindungsgemäßen Vorrichtung die nötige Durchflussgeschwindigkeit zur Aufreinigung des Proteins mittels Zentrifugation erzielt wird, ist ein Anlegen von Unterdruck bzw. Vakuum nicht nötig, wodurch das proteinhaltige Fluid geschont wird. Zudem vereinfacht dies den Aufbau der erfindungsgemäßen Proteinreinigungsvorrichtung, da die Vorrichtung kein Anschlusssystem zur Erzeugung von Unterdruck aufweisen muss. Aufgrund der kompakten, modularen Bauart der erfindungsgemäßen Vorrichtung mit einem Membranadsorber anstelle einer Chromatographiesäule kann ein deutlich größeres Volumen an proteinhaltigem Fluid pro Zentrifugationsvorgang aufgereinigt werden als bei bisher bekannten Vorrichtungen, die zur Zentrifugation geeignet sind. Zudem kann die poröse Membran (5) der erfindungsgemäßen Vorrichtung vorteilhafterweise trocken und bei Raumtemperatur gelagert werden, ohne dass ein Stabilitätsverlust eintritt. Darüber hinaus kann durch die einfache Sterilisierbarkeit der erfindungsgemäßen Vorrichtung eine Verkeimung ohne hohen Aufwand vermieden werden. Die erfindungsgemäße Vorrichtung und das erfindungsgemäße Verfahren erlauben es somit vorteilhafterweise, schnell, effektiv, zuverlässig und steril Protein aus einem proteinhaltigen Fluid aufzureinigen.

### Bezugszeichenliste

- 1: zylinderförmige Vorfiltereinheit
- 2: zylinderförmige Membranadsorbereinheit
- 3: zylinderförmiger Auffangbehälter
- 4: Größenausschlussfilter
- 5: poröse Membran
- 6: Endkappe

Figur 1 zeigt die Bauteile einer erfindungsgemäßen Vorrichtung als Einzelteile.
Figur 2 zeigt eine erfindungsgemäße Vorrichtung im "zusammengesteckten Zustand".
Figur 3 definiert die Begriffe "Innenwandzylinderdurchmesser", "Außenwandzylinderdurchmesser" und "Länge" der Bauteile der erfindungsgemäßen Vorrichtung.

## Patentansprüche

1. Vorrichtung zum Aufreinigen von Protein aus einem proteinhaltigen Fluid, umfassend
eine zylinderförmige Vorfiltereinheit (1), die mindestens einen Größenausschlussfilter (4) aufweist;
eine zylinderförmige Membranadsorbereinheit (2), die mindestens eine poröse Membran (5) mit funktionellen Bestandteilen aufweist, die das aufzureinigende Protein adsorbieren; und
einen zylinderförmigen Auffangbehälter (3);
wobei der Auffangbehälter (3), die Membranadsorbereinheit (2) und die Vorfiltereinheit (1) derart ausgestaltet sind, dass der Auffangbehälter (3) zur Aufnahme der Membranadsorbereinheit (2) geeignet ist und die Membranadsorbereinheit (2) zur Aufnahme der Vorfiltereinheit (1) geeignet ist.

2. Vorrichtung nach Anspruch 1, wobei die Vorfiltereinheit (1) zur Aufnahme von 0,5 bis 25 mL Fluid geeignet ist und der Auffangbehälter (3) zur Aufnahme von 1 bis 50 mL Fluid geeignet ist.

3. Vorrichtung nach Anspruch 1 oder 2, wobei der mindestens eine Größenausschlussfilter (4) an einem Ende der zylinderförmigen Vorfiltereinheit (1) angeordnet ist.

4. Vorrichtung nach einem der Ansprüche 1 bis 3, wobei die funktionellen Bestandteile der mindestens einen porösen Membran (5) der Membranadsorbereinheit (2) ausgewählt sind aus Ni²⁺, Cu²⁺, Protein A, Streptavidin oder Derivate, und Amylose.

5. Vorrichtung nach einem der Ansprüche 1 bis 4, wobei die poröse Membran (5) der Membranadsorbereinheit (2) aus zwei oder mehr Membranlagen

6. Vorrichtung nach einem der Ansprüche 1 bis 5, wobei die poröse Membran (5) an einem Ende der Membranadsorbereinheit (2) angeordnet ist.

7. Vorrichtung nach einem der Ansprüche 1 bis 6, weiter umfassend ein Flussbeschränkungsmittel, das derart ausgestaltet ist, dass bei Zentrifugierung der Vorrichtung die Verweildauer des proteinhaltigen Fluids in der porösen Membran (5) erhöht wird.

8. Vorrichtung nach Anspruch 7, wobei das Flussbeschränkungsmittel benachbart zur porösen Membran (5) der Membranadsorbereinheit (2) angeordnet ist.

9. Vorrichtung nach Anspruch 7 oder 8, wobei das Flussbeschränkungsmittel mindestens eine Membran ist, die eine geringere Porosität als die poröse Membran (5) der Membranadsorbereinheit (2) aufweist.

10. Verfahren zur Aufreinigung von Protein aus einem proteinhaltigen Fluid, umfassend die Schritte
(a) Bereitstellen der Vorrichtung nach einem der Ansprüche 1 bis 9, wobei sich die Vorfiltereinheit (1) in der Membranadsorbereinheit (2) befindet und sich die Membranadsorbereinheit (2) in dem Auffangbehälter (3) befindet;
(b) Befüllen der Vorfiltereinheit (1) mit proteinhaltigem Fluid;
(c) Zentrifugieren der Vorrichtung, wobei die poröse Membran (5) verblockende Bestandteile des proteinhaltigen Fluids von dem mindestens einen Größenausschlussfilter (4) zurückgehalten werden, das zu reinigende Protein an der porösen Membran (5) der Membranadsorbereinheit (2) adsorbiert und sich ein Filtrat in dem Auffangbehälter (3) sammelt;
(d) Verwerfen der Vorfiltereinheit (1) und des im Auffangbehälter (3) befindlichen Filtrats;
(f) Befüllen der Membranadsorbereinheit (2) mit Elutionspufferlösung;
(g) Eluieren des an der porösen Membran (5) adsorbierten Proteins mit der Elutionspufferlösung in den Auffangbehälter (3) zum Erhalt des aufgereinigten Proteins.

11. Verfahren nach Anspruch 10, weiter umfassend einen Schritt (e) zwischen den Schritten (d) und (f),
(e) Befüllen der Membranadsorbereinheit (2) mit einer Waschpufferlösung, Zentrifugieren der Vorrichtung ohne Vorfiltereinheit (1), so dass sich die Waschpufferlösung in dem Auffangbehälter (3) sammelt und anschließendes Verwerfen der im Auffangbehälter (3) befindlichen Waschpufferlösung.

12. Verfahren nach Anspruch 10 oder 11, wobei Schritt (g) unter Zentrifugation erfolgt.

13. Verfahren nach einem der Ansprüche 10 bis 12, wobei sämtliche Schritte des Verfahrens unter Standardatmosphärendruck durchgeführt werden.

14. Verwendung der Vorrichtung nach einem der Ansprüche 1 bis 9 zur Aufreinigen von Protein aus einem proteinhaltigen Fluid.

15. Verwendung nach Anspruch 14, wobei das zu reinigende Protein ein His-getaggtes Protein ist.
